# EUROPEAN PATENT APPLICATION

(11) **EP 2 251 002 A1**
(43) Date of publication of application: **17.11.2010**
(21) Application number: 09708223.4
(22) Date of filing: 06.02.2009
(51) Int. Cl.: A61K 8/55, A61Q 7/00

(54) **HAIR GROWING COSMETIC**

(30) Priority: 06.02.2008 JP 2008026473
(71) Applicant: Showa Denko K.K., Tokyo 105-8518 (JP)
(72) Inventor: AOKI, Hirobumi, Chiba-shi Chiba 267-0056 (JP); KAMACHI, Harumi, Chiba-shi Chiba 267-0056 (JP)
(74) Representative: Strehl Schübel-Hopf & Partner
(86) International application number: PCT/JP2009/052025
(87) International publication number: WO 2009/099167

(57) **Abstract**

It is an object of the present invention to provide a hair nourishing cosmetic which allows an antioxidative action of ubiquinone to contribute to hair nourishment. The hair nourishing cosmetic of the present invention comprises a ubiquinone derivative represented by the following formula (1) and/or its salt, 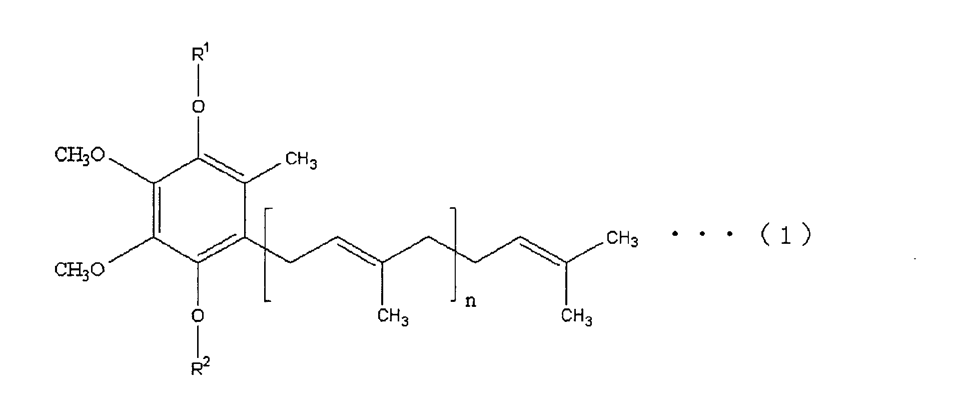 wherein R¹ and R² are each independently a hydrogen atom or a phosphoric acid group, at least one of R¹ and R² is a phosphoric acid group, and n is an integer of 1 to 9.

## Description

### TECHNICAL FIELD

The present invention relates to a hair nourishing cosmetic comprising a ubiquinone derivative and/or its salt, which exerts an excellent hair growing effect and an excellent hair loss prevention effect.

### BACKGROUND ART

It is said that one cause of androgenetic alopecia is an excess action of male hormone, and it is also said that other various phenomena, such as poor blood circulation in a scalp, lowering of activity of hair matrix cells, enlargement of sebaceous glands and disorders of a scalp due to oxidation of sebum, take part in that complicatedly.

Under such background, hair nourishing cosmetics containing amino acid and vitamins which are blood circulation promotion substances and nutritional ingredients for hair are known. Not only such hair nourishing cosmetics but also a large number of medical hair nourishing agents and hair nourishing cosmetics in which ingredients having an action of suppressingmale hormone or ingredients having an effect of preventing enlargement of sebaceous glands were blended have been proposed.

Moreover, there have been proposed a large number of hair nourishing/hair growing agents, such as a hair growing agent composition containing a fatty acid or an alcohol having carbon atoms of an odd number, hydroxypropyl alcohol and a lower alcohol (patent document 1), a hair restorer containing a certain kind of carboxyl betaine (patent document 2), a hair nourishing cosmetic containing an acidic mucopolysaccharide and a hop extract (patent document 3), a hair nourishing cosmetic containing acetyl carnitine and its salt (patent document 4) and an anti-hair loss hair dressing containing an *Abelmoschus manihot* extract (patent document 5).

On the other hand, ubiquinone, i.e., a natural ingredient that is so-called coenzyme Q, is an important substance in vivo as an antioxidative substance, and is widely utilized for foods, cosmetics, etc. Ubiquinone itself, however, is hardly-soluble in water and has properties such as high crystallizability, so that formulation using it is difficult. And it is unknown that ubiquinone contributes to hair nourishment. Furthermore, any of the hair nourishing cosmetics described above does not utilize such ubiquinone.
Patent document 1: Japanese Patent Laid-Open Publication No. 1993-58850
Patent document 2: Japanese Patent Laid-Open Publication No. 1993-139936
Patent document 3: Japanese Patent Laid-Open Publication No. 1993-170625
Patent document 4: Japanese Patent Laid-Open Publication No. 2000-016920
Patent document 5: Japanese Patent Laid-Open Publication No. 1986-249913

### DISCLOSURE OF THE INVENTION

### PROBLEM TO BE SOLVED BY THE INVENTION

Accordingly, it is an object of the present invention to provide a hair nourishing cosmetic which allows an antioxidative action of ubiquinone to contribute hair nourishment.

### MEANS TO SOLVE THE PROBLEM

The present inventors have found that a ubiquinone derivative having a specific structure and/or its salt is excellent in affinity for the skin, and by applying it to the skin, excellent hair nourishing effect is obtained. Accordingly, they have found that the above object is attained by adding a specific ubiquinone derivative and/or its salt to a hair-nourishing cosmetic. Thus, the present inventors have accomplished the present invention.

That is to say, the present invention relates to, for example, the following matters [1] to [3].
[1] A hair nourishing cosmetic comprising a ubiquinone derivative represented by the following formula (1) and/or its salt:

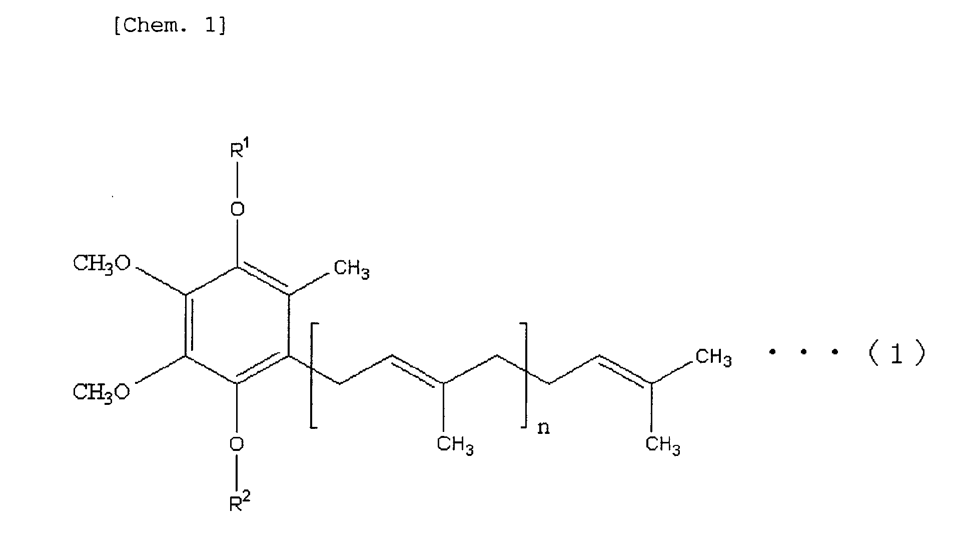

wherein R¹ and R² are each independently a hydrogen atom or a phosphoric acid group, at least one of R¹ and R² is a phosphoric acid group, and n is an integer of 1 to 9.
[2] The hair nourishing cosmetic as stated in [1], wherein the ubiquinone derivative and/or its salt is contained in a concentration of 0.0005 to 50% by mass.
[3] The hair nourishing cosmetic as stated in [1] or [2], wherein in the formula (1), both of R¹ and R² are phosphoric acid groups, and n is an integer of 5 to 9.

### EFFECT OF THE INVENTION

According to the present invention, a hair nourishing cosmetic to which ubiquinone exhibiting no favorable formulation properties does not need to be directly added and which makes the most of an antioxidative action equivalent or superior to that of ubiquinone can be provided. Moreover, the hair nourishing cosmetic of the present invention exerts an excellent hair growing effect and an excellent hair loss prevention effect.

### BRIEF DESCRIPTION OF THE DRAWING

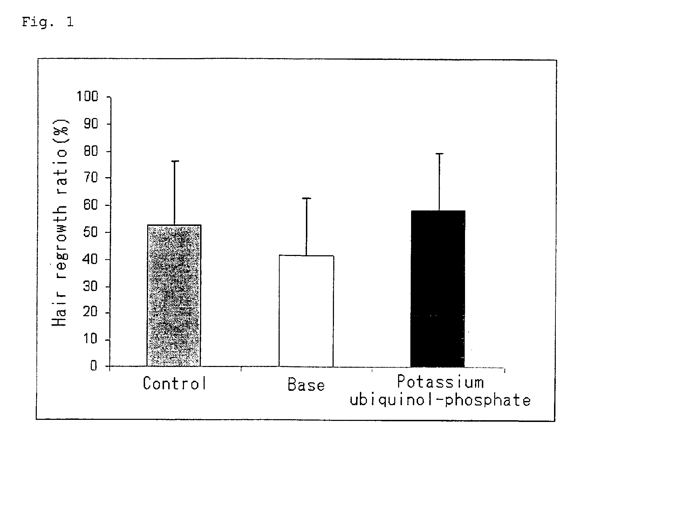

Fig. 1 shows hair regrowth ratios in Test Example 3.

### BEST MODE FOR CARRYING OUT THE INVENTION

The constitutions of the present invention are described in detail hereinafter.

The hair nourishing cosmetic according to the present invention comprises a ubiquinone derivative represented by the following formula (1) and/or its salt.

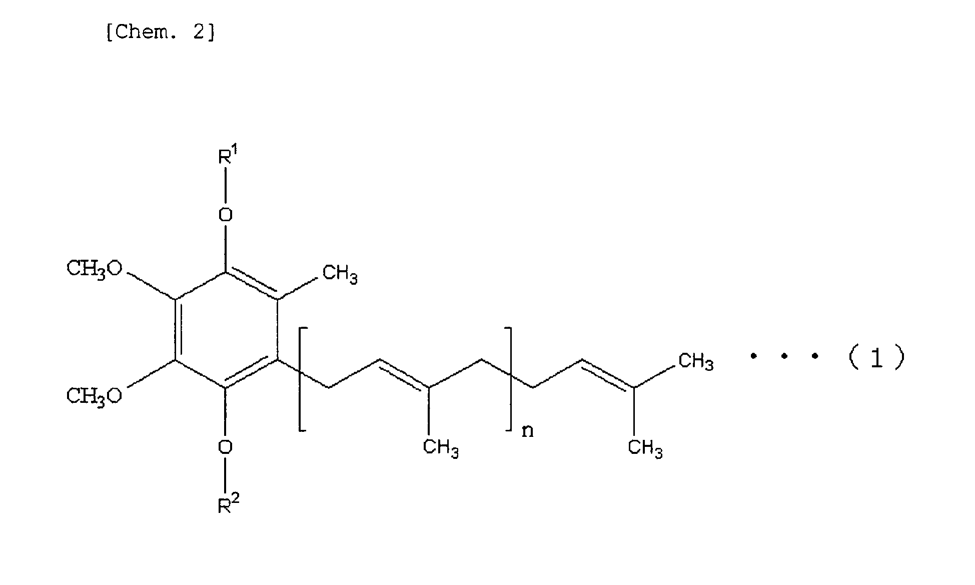

In the above formula, R¹ and R² are each independently a hydrogen atom or a phosphoric acid group. At least one of R¹ and R² is a phosphoric acid group, and it is preferable that both of them are phosphoric acid groups. n is usually an integer of 1 to 9, and from the viewpoint of retention of practical solubility and affinity for the skin with a good balance, n is preferably an integer of 5 to 9, and more preferably 9.

Such a ubiquinone derivative modified with a phosphoric acid group (also referred to as a "ubiquinone derivative" simply hereinafter) can be obtained by a conventional process. For example, quinone in a ubiquinone prepared in advance is reduced to give a quinol type, and addition of a phosphoric acid to the hydroxyl group is further carried out in a conventional manner to synthesize a ubiquinone derivative. Such a process is disclosed in U.S. Patent No. 2, 962, 519, etc. in detail.

In the present invention, a salt of the above ubiquinone derivative modified with a phosphoric acid group may be used. The salt of the ubiquinone derivative means a substance wherein an anion of the phosphoric acid group of the ubiquinone derivative and a cation form a salt. Examples of such cations include sodium ion, potassium ion, calcium ion, magnesium ion, zinc ion and ammonium ion. Of these, sodium ion and potassium ion are more preferable from the viewpoint of blendability with a hair nourishing cosmetic.

Such ubiquinone derivatives are used singly or as mixtures with salts of ubiquinone derivatives. The ubiquinone derivatives and salts thereof are also referred to as a "ubiquinone derivative group" generically.

The ubiquinone derivative group has water solubility markedly higher than that of ubiquinones. For example, the solubility of ubiquinone having 10 side-chain isoprene units in water is less than 100 ppm at normal temperature, but the solubility of a diphosphate thereof exceeds 1% by mass. That is to say, the ubiquinone derivative group exhibits water solubility easily exceeding 100 times the water solubility of ubiquinone. Therefore, an aqueous formulation which is stable and contains the ubiquinone derivative group in a high concentration can be easily prepared, and there is no fear of precipitation and the like. Moreover, the radical elimination ability of the ubiquinone derivative group in an aqueous system is higher than that of ubiquinone, and a hair nourishing cosmetic imparted with higher antioxidative ability can be realized.

When the ubiquinone derivative group is incorporated into human skin cells, it is converted into the corresponding ubiquinone or ubiquinol. That is to say, it is considered that the ubiquinone derivative group administered into cells is rapidly dephosphorylated in the cells and becomes ubiquinone through ubiquinol that is a primary product thereof. It is also considered that ubiquinone generated by oxidation from ubiquinol liberated in the cells undergoes turnover in the cells to thereby exhibit an antioxidative effect that the capacity in the oxidation reduction process is enhanced.

It is considered that the antioxidative ability of the ubiquinone derivative group inhibits excessive oxidation of sebum at the root of hair to thereby effectively prevent hair loss attributable to accumulation of oxidized sebum. Therefore, the ubiquinone derivative group is presumed to exert a favorable effect as an ingredient added to hair nourishing cosmetics which are required to have a hair growing effect and a hair loss prevention effect.

The amount of ubiquinone added to a hair nourishing cosmetic is regulated to not more than 0.03% by mass at present in accordance with the provisions by the Ministry of Health, Labour and Welfare. Even in such an amount, efficacy of ubiquinone has been recognized, and it has been widely used. Under such background, the concentration of ubiquinone acid derivative group in the hair nourishing cosmetic does not need to be specifically restricted, and it has only to be determined according to the magnitude of the desired hair growing effect and the hair loss prevention effect. When the concentration of the ubiquinone derivative group is not less than about 0.0005% by mass, preferably not less than 0.001% by mass, more preferably not less than 0.01% by mass, efficacy of ubiquinone and ubiquinol can be substantially given. There is no specific restriction on the maximum concentration of the ubiquinone derivative group, but a practical aqueous formulation exhibiting the effect of the present invention more effectively is a hair nourishing cosmetic having a ubiquinone derivative group concentration of not more than 50% by mass, preferably not more than 20% by mass, and more preferably not more than 10% by mass. However, a formulation provided as a solid formulation, a concentrated formulation or a dispersion formulation each having a ubiquinone derivative group content of not less than 50% by mass may be prepared so that the formulation should have the above concentration when it is used.

In addition to the ubiquinone derivative group, ingredients generally used for hair nourishing cosmetics may be added to the hair nourishing cosmetic of the present invention in amounts not detrimental to the effect of the present invention.

The ingredients which can be added are not specifically restricted, but for example, such ingredients as given below can be mentioned.

hydrocarbons, such as ozokerite, α-olefin oligomer, light isoparaffin, light liquidisoparaffin, squalene, squalane, synthetic squalane, vegetable squalane, ceresin, paraffin, polyethylene powder, polybutene, microcrystalline wax, liquid isoparaffin, liquid paraffin, mineral oil and vaseline;

natural fats and oils, e.g., natural waxes, such as jojoba oil, carnauba wax, candelilla wax, rice wax, shellac, lanoline, mink tallow wax, spermaceti, sugar cane wax, sperm whale oil, beeswax and montan wax, avocado oil, almond oil, olive oil, extra virgin olive oil, sesame oil, rice bran oil, rice oil, rice germ oil, corn oil, soybean oil, corn oil, persic oil, palm kernel oil, palm oil, castor oil, grape seed oil, cottonseed oil, coconut oil, hydrogenated coconut oil, beef tallow, hydrogenated oil, horse oil, mink oil, egg yolk oil, egg yolk fatty oil, rose hips oil, candlenut oil, evening primrose oil, wheat germ oil, peanut oil, camellia oil, sasanqua oil, cacao butter, Japan wax, beef bone fat, beef foot oil, hog fat, horse fat, mutton tallow, shea butter, macademia nut oil and meadowfoam seed oil;

fatty acids, such as lauric acid, myristic acid, palmitic acid, stearic acid, behenic acid, oleic acid, isostearic acid, 12-hydroxystearic acid, undecylenic acid and coconut fatty acid;

higher monoalcohols, such as isostearyl alcohol, octyl dodecanol, hexyl decanol, cholesterol, phytosterol, lauryl alcohol, myristyl alcohol, cetanol, stearyl alcohol, oleyl alcohol, behenyl alcohol and cetostearyl alcohol;

alkyl glyceryl ethers, such as batyl alcohol, chimyl alcohol, selachyl alcohol and isostearyl glyceryl ether;

esters, such as isopropyl myristate, butyl myristate, isopropyl palmitate, ethyl stearate, butyl stearate, ethyl oleate, ethyl linoleate, isopropyl linoleate, cetyl caprylate, hexyl laurate, isooctyl myristate, decyl myristate, myristyl myristate, cetyl myristate, octadecyl myristate, cetyl plamitate, stearyl stearate, decyl oleate, oleyl oleate, cetyl ricinoleate, isostearyl laurate, isotridecyl myristate, isocetyl myristate, isostearyl myristate, octyldodecyl myristate, 2-ethylhexyl palmitate, isocetyl palmitate, isostearyl palmitate, 2-ethylhexyl stearate, isocetyl stearate, isodecyl oleate, octyldodecyl oleate, oxtyldodecyl ricinoleate, ethyl isostearate, isopropyl isostearate, cetyl 2-ethylhexanoate,
cetostearyl 2-ethylhexanoate, stearyl 2-ethylhexanoate, hexyl isostearate, ethylene glycol dioctanoate, ethylene glycol dioleate, propylene glycol dicaprylate, propylene glycol dicaprylate/dicaprate, propylene glycol dicaprate, propylene glycol dioleate, neopentyl glycol dicaprate, neopentyl glycol dioctanoate, glyceryl tricaprylate, glyceryl tri-2-ethylhexanoate, caprylic/capric acid triglyceride, caprylic/capric/stearic acid triglyceride, glyceryl triundecylate, glyceryl triisopalmitate, glyceryl triisostearate, trimethylolpropane tri-2-ethylhexanoate, trimethylolpropane triisostearate, pentaerythrityl tetra-2-ethylhexanoate, pentaerythrityl tetramyristate, pentaerythrityl tetraisostearate, diglyceryl tetraisostearate, octyldodecyl neopentanoate, isocetyl octanoate, isostearyl octanoate, 2-ethylhexyl isopelargonate, hexyldecyl dimethyloctanoate, octyldodecyl dimethyloctanoate, 2-ethylhexyl isopalmitate, isocetyl isostearate, isostearyl isostearate, octyldodecyl isostearate, lauryl lactate, myristyl lactate, cetyl lactate, octyldodecyl lactate, triethyl citrate, acetyltriethyl citrate, acetyltributyl citrate, trioctyl citrate, triisocetyl citrate, trioctyldodecyl citrate, diisostearyl malate, 2-ethylhexyl hydroxystearate, di-2-ethylhexyl succinate, diisopropyl adipate,
diisobutyl adipate, dioctyl adipate, diheptylundecyl adipate, diethyl sebacate, diisopropyl sebacate, dioctyl sebacate, cholesteryl stearate, cholesteryl isostearate, cholesteryl hydroxystearate, cholesteryl oleate, dihydrocholesteryl oleate, phytosteryl isostearate, phytosteryl oleate, isocetyl 12-stearoylhydroxystearate, stearyl 12-stearoylhydroxystearate, isostearyl 12-stearoylhydroxystearate, polyoxyethylene(3) polyoxypropylene(1) cetyl ether acetate, polyoxyethylene(3) polyoxypropylene(1) isocetyl ether acetate, isononyl isononanoate, octyl isononanoate, tridecyl isononanoate and isotridecyl isononanoate;

silicone oils, such as methylpolysiloxane, methylphenylpolysiloxane, methylhydrogenpolysiloxane, methylcyclopolysiloxane, octamethylcyclotetrasiloxane, decamethylcyclopentasiloxane, dodecamethylcyclohexasiloxane, octamethyltrisiloxane, decamethyltetrasiloxane, tetradecamethylhexasiloxane, highly polymerized methylpolysiloxane, dimethylsiloxane/methyl(polyoxyethylene)siloxane/methyl(polyoxyp ropylene)siloxane copolymer,
dimethylsiloxane/methyl(polyoxyethylene)siloxane copolymer, dimethylsiloxane/methyl(polyoxypropylene)siloxane copolymer, dimethylsiloxane/methylcetyloxysiloxane copolymer, dimethylsiloxane/methylstearoxysiloxane copolymer, polyether modified silicone, alcohol modified silicone, alkyl modified silicone and amino modified silicone;

high-molecular weight substances, such as sodium alginate, carrageenan, agar, furcelleran, guar gum, quince seed, konjakmannan, tamarind gum, tara gum, dextrin, starch, locust bean gum, gum arabic, gum ghatti, karaya gum, gum tragacanth, arabinogalactan, pectin, quince, chitosan, starch, curdlan, xanthane gum, gellan gum, cyclodextrin, dextran, pullulan, microcrystalline cellulose, methyl cellulose, ethyl cellulose, hydroxyethyl cellulose, hydroxypropyl cellulose, hydroxypropylmethyl cellulose, carboxymethyl cellulose, carboxystarch, cationized cellulose, starch phosphoric acid ester, cationized guar gum, carboxymethyl hydroxypropylated guar gum, hydroxypropylated guar gum, albumin, casein, gelatin, sodium polyacrylate, polyacrylic acid amide, carboxyvinyl polymer, polyethyleneimine, highly polymerized polyethylene glycol, polyvinyl alcohol, polyvinyl pyrrolidone, polyvinyl ether, polyacrylamide, acrylic acid copolymer, methacrylic acid copolymer, maleic acid copolymer, vinylpyridine copolymer, ethylene/acrylic acid copolymer, vinyl pyrrolidone-based polymer, vinyl alcohol/vinyl pyrrolidone copolymer, nitrogen substituted acrylamide-based polymer, amino modified silicone, cationized polymer, dimethylacrylammonium-based polymer, acrylic acid-based anionic polymer, methacrylic acid-based anionic polymer, modified silicone, alkyl(C10-30) acrylate methacrylate copolymer and polyoxyethylene/polyoxypropylene copolymer;

lower monoalcohols, such as ethanol, isopropyl alcohol, 1-butanol, 2-butanol and benzyl alcohol;

polyhydric alcohols, such as ethylene glycol, diethylene glycol, polyethylene glycol, propylene glycol, polypropylene glycol, glycerin, diglycerin, polyglycerin, 1,3-butanediol, triethylene glycol, dipropylene glycol, 3-methyl-1,3-butanediol, 1,2-pentanediol, 1,4-pentanediol, 1,5-pentanediol, 2,4-pentanediol, 2-methyl-2,4-pentanediol, 3-methyl-1,5-pentanediol, 1,2-hexanediol and 1,6-hexanediol;

anionic surface active agents, such as potassium cocoate, sodium cocoate, triethanolamine cocoate, potassium laurate, sodium laurate, triethanolamine laurate, potassium myristate, sodium myristate, isopropanolamine myristate, potassium palmitate, sodium palmitate, isopropanolamine palmitate, potassium stearate, sodium stearate, triethanolamine stearate, potassium oleate, sodium oleate, sodium castor oil fatty acid, zinc undecylenate, zinclaurate, zincmyristate, magnesiummyristate, zincpalmitate, zincstearate, calcium stearate, magnesium stearate, aluminum stearate, calcium myristate, magnesium myristate, aluminum dimyristate, aluminum isostearate, polyoxyethylene lauryl ether acetic acid, sodium polyoxyethylene lauryl ether acetate, polyoxyethylene tridecyl ether acetic acid, sodium polyoxyethylene tridecyl ether acetate, sodium stearoyl lactate, sodium isostearoyl lactate, sodium lauroyl sarcosinate, cocoyl sarcosine, sodium N-cocoyl sarcosinate, triethanolamine N-cocoyl sarcosinate, lauroyl sarcosine,
potassium lauroyl sarcosinate, triethanolamine lauroyl sarcosinate, oleoyl sarcosine, sodium myristoyl sarcosinate, sodium stearoyl glutamate, cocoyl glutamic acid, potassium cocoyl glutamate, sodium cocoyl glutamate, triethanolamine cocoyl glutamate, lauroyl glutamic acid, potassium lauroyl glutamate, sodium lauroyl glutamate, triethanolamine lauroyl glutamate, myristoyl glutamic acid, potassium myristoyl glutamate, sodium myristoyl glutamate, stearoyl glutamic acid, potassium stearoyl glutamate, disodium stearoyl glutamate, sodium hydrogenated tallow glutamate, sodium cocoyl glutamate/hydrogenated tallow glutamate, sodium N-methyl N-(1-oxococonut alkyl) β-alanine, lauroyl methyl alanine, sodium lauroyl methyl alaninate, triethanolamine lauroyl methyl alaninate, sodium myristoyl methyl alaninate, sodium lauroyl methyl taurate,
potassium N-cocoyl N-methyl taurate, sodium N-cocoyl N-methyl taurate, magnesium cocoyl methyl taurate, sodium myristoyl methyl taurate, sodium palmitoyl N-methyl taurate, sodium stearoyl methyl taurate, sodium oleoyl methyl taurate, sodium alkanesulfonate, sodium tetradecenesulfonate, di(2-ethylhexyl)sodium sulfosuccinate, disodium monolauryl sulfosuccinate, sodium cocoyl ethyl ester sulfonate, sodium lauryl sulfate, triethanolamine lauryl sulfate, sodium cetyl sulfate, triethanolamine alkyl(11,13,15) sulfate, sodium alkyl(12,13) sulfate, triethanolamine alkyl(12,13) sulfate, ammonium alkyl(12,14,16) sulfate, diethanolamine alkyl(12-13) sulfate, triethanolamine alkyl(12-14) sulfate, triethanolamine alkyl(12-15) sulfate, magnesium·triethanolamine coco sulfate, ammonium lauryl sulfate, potassium lauryl sulfate, magnesium lauryl sulfate,
monoethanolamine lauryl sulfate, diethanolamine lauryl sulfate, sodiummyristyl sulfate, sodiumstearyl sulfate, sodiumoleyl sulfate, triethanolamine oleyl sulfate, sodium polyoxyethylene lauryl ether sulfate, triethanolamine polyoxyethylene lauryl ether sulfate,
sodium polyoxyethylene(1) alkyl(11,13,15) ether sulfate, triethanolamine polyoxyethylene(1) alkyl(11,13,15) ether sulfate, sodium polyoxyethylene(3) alkyl(11-15) ether sulfate, sodium polyoxyethylene(2) alkyl(12,13) ether sulfate, sodium polyoxyethylene(3) alkyl(12-14) ether sulfate, sodium polyoxyethylene(3) alkyl(12-15) ether sulfate, sodium polyoxyethylene(2) lauryl ether sulfate, sodium polyoxyethylene(3) myristyl ether sulfate, higher fatty acid alkanolamide sulfuric acid ester sodium salt, lauryl monophosphate, sodium lauryl phosphate, potassium cetyl phosphate,
diethanolamine cetyl phosphate, polyoxyethylene oleyl ether phosphate, polyoxyethylene lauryl ether phosphate, sodium polyoxyethylene lauryl ether phosphate, polyoxyethylene cetyl ether phosphate, sodium polyoxyethylene cetyl ether phosphate, polyoxyethylene stearyl ether phosphate, polyoxyethylene oleyl ether phosphate, sodium polyoxyethylene oleyl ether phosphate, polyoxyethylene alkylphenyl ether phosphate, sodium polyoxyethylene alkylphenyl ether phosphate, triethanolamine polyoxyethylene alkylphenyl ether phosphate, polyoxyethylene octyl ether phosphate, polyoxyethylene(10) alkyl(12,13) ether phosphate, polyoxyethylene alkyl(12-15) ether phosphate, polyoxyethylene alkyl(12-16) ether phosphate, triethanolamine polyoxyethylene lauryl ether phosphate and diethanolamine polyoxyethylene oleyl ether phosphate;

cationic surface active agents, such as dioctylamine, dimethylstearylamine, trilaurylamine, N-[2-(diethylamino)ethyl] octadecanamide, lauryltrimethylammonium chloride, cetyltrimethylammonium chloride, cetyltrimethylammonium bromide, cetyltrimethylammonium saccharinate, stearyl trimethyl ammonium chloride, alkyl(20-22) trimethylammonium chloride, lauryltrimethylammonium bromide, alkyl(16,18) trimethylammonium chloride, stearyl trimethyl ammonium bromide, stearyl trimethyl ammonium saccharinate, alkyl(28) trimethylammonium chloride, di(polyoxyethylene) oleyl methyl ammonium chloride (2EO), dipolyoxyethylene stearyl methyl ammonium chloride, polyoxyethylene(1) polyoxypropylene(25) diethyl methyl ammonium chloride, tri(polyoxyethylene) stearyl ammonium chloride (5EO), distearyldimethylammonium chloride, dialkyl(12-15) dimethyl ammonium chloride, dialkyl(12-18) dimethyl ammonium chloride, dialkyl(14-18) dimethyl ammonium chloride, dicocoyl dimethyl ammonium chloride, dicetyldimethylammonium chloride, isostearyl lauryl dimethyl ammonium chloride, benzalkonium chloride, myristyl dimethyl benzyl ammonium chloride, lauryl dimethyl (ethylbenzyl) ammonium chloride, stearyldimethylbenzylammonium chloride, lauryl pyridinium chloride, cetylpyridinium chloride, N-(lauroyl colamino formyl methyl)pyridinium chloride, N-(stearoyl colamino formyl methyl)pyridinium chloride, alkylisoquinolinium bromide, methyl benzethonium chloride and benzethonium chloride;

amphoteric surface active agents, such as 2-alkyl-N-carboxymethyl-N-hydroxyethylimidazolinium betaine, alkyldiaminoethylglycine hydrochloride, sodium lauryldiaminoethylglycinate, sodium 2-undecyl-1-hydroxyethyl imidazoliium betaine, undecyl-N-carboxymethylimidazoliium betaine, disodium N-cocoyl-N-carboxyethyl-N-hydroxyethylethylenediamine, disodium N-cocoyl-N-carboxyethoxyethyl-N-carboxylethyl ethylenediamine, disodium N-cocoyl-N-carboxymethoxyethyl-N-carboxymethylethylenediamine, sodium laurylaminopropionate, sodium laurylaminodipropionate, triethanolamine laurylaminopropionate, sodium N-palm oil fatty acid acyl-N-carboxyethyl-N-hydroxyethylethylenediamine, lauryldimethylaminoacetic acid betaine, cocoalkyl dimethyl glycine, stearyldimethylaminoacetic acid betaine, sodium stearyl dimethyl glycine, cocoyl amide propyldimethyl glycine, palm oil amide propyl dimethyl glycine, lauric acid amide propylacetic acid betaine, propyl betaine ricinoleate amide, stearyl dihydroxyethyl glycine and lauryl hydroxy sulfobetaine;

nonionic surface active agents, such as polyoxyethylene(10) alkyl(12,13) ether, polyoxyethylene lauryl ether, polyoxyethylene cetyl ether, polyoxyethylene stearyl ether, polyoxyethylene oleyl ether, polyoxyethylene (3, 7, 12) alkyl (12-14) ether, polyoxyethylene tridecyl ether, polyoxyethylene myristyl ether, polyoxyethylene-sec-alkyl(14) ether, polyoxyethylene isocetyl ether, polyoxyethylene cetostearyl ether, polyoxyethylene(2,10,20) isostearyl ether, polyoxyethylene oleylcetyl ether, polyoxyethylene(20) arachyl ether, polyoxyethylene octyldodecyl ether, polyoxyethylene behenyl ether, polyoxyethylene octylphenyl ether, polyoxyethylene nonylphenyl ether, polyoxyethylene dinonylphenyl ether, polyoxyethylene(1) polyoxypropylene(1,2,4,8) cetyl ether, polyoxyethylene(5) polyoxypropylene(1,2,4,8) cetyl ether, polyoxyethylene(10) polyoxypropylene(1,2,4,8) cetyl ether, polyoxyethylene(20) polyoxypropylene(1,2,4,8) cetyl ether, polyoxyethylene polyoxypropylene lauryl ether, polyoxyethylene(3) polyoxypropylene(34) stearyl ether, polyoxyethylene(4) polyoxypropylene(30) stearyl ether, polyoxyethylene(34) polyoxypropylene(23) stearyl ether, polyoxyethylene polyoxypropylene cetyl ether, polyoxyethylene polyoxypropylene decyltetradecyl ether, polyethylene glycol monolaurate, ethylene glycol monostearate, polyethylene glycol monostearate, polyethylene glycol monooleate, ethylene glycol fatty acid ester, ethylene glycol monostearate (self-emulsifying), diethylene glycol laurate, polyethylene glycol myristate, polyethylene glycol palmitate, diethylene glycol stearate, polyethylene glycol(2) monostearate (self-emulsifying), polyethylene glycol isostearate, ethylene glycol dioctanoate, diethylene glycol dilaurate, polyethylene glycol dilaurate, polyethylene glycol(150) dipalmitate, ethylene glycol distearate,
diethylene glycol distearate, polyethylene glycol distearate, ethylene glycol dioleate, polyethylene glycol dioleate, polyethylene glycol diricinoleate, polyoxyethylene(20) sorbitan monolaurate, polyoxyethylene(20) sorbitan monopalmitate, polyoxyethylene(6) sorbitan monostearate, polyoxyethylene(20) sorbitan monostearate, polyoxyethylene(20) sorbitan tristearate, polyoxyethylene(6) sorbitan monooleate, polyoxyethylene(20) sorbitan monooleate, polyoxyethylene(20) sorbitan trioleate, polyoxyethylene(20) sorbitan monococoate, polyoxyethylene (10-80) sorbitan monolaurate, polyoxyethylene sorbitan tristearate, polyoxyethylene (20) sorbitan isostearate, polyoxyethylene(150) sorbitan tristearate, polyoxyethylene castor oil, polyoxyethylene hydrogenated castor oil, polyoxyethylene(10) hydrogenated castor oil, polyoxyethylene(20) hydrogenated castor oil, polyoxyethylene (40) hydrogenated castor oil, polyoxyethylene(50) hydrogenated castor oil, polyoxyethylene(60) hydrogenated castor oil, lipophilic glyceryl monostearate, lipophilic glyceryl monooleate, glyceryl monostearate (self-emulsifying), glyceryl cocoate, glyceryl laurate,
glyceryl myristate,glycerylisostearate,glycerylricinoleate, glyceryl monohydroxystearate, glyceryl oleate, glyceryl linoleate, glyceryl erucate, glyceryl behenate, wheat germ fatty acid glycerides, safflower oil fatty acid glycerin monoester, hydrogenated soy glyceride, saturated fatty acid glycerides, cottonseed oil glyceride, glyceryl monoisostearate monomyristate, glyceryl monotallowate, glyceryl monolanolate, glyceryl sesquioleate, glyceryl distearate, glyceryl diisostearate, glyceryl diarachate, sorbitan monolaurate, sobitan monopalmitate, sorbitan monostearate, sorbitan monoisostearate, sorbitan monooleate, sorbitan sesquistearate, sorbitan sesquioleate, sorbitan tristearate, sorbitan trioleate, sobitan monococoate, sorbitan isostearate, sorbitan sesquiisostearate,
sorbitan distearate, diglyceryl isopalmitate, poly(4-10)glyceryl monolaurate, poly(10)glyceryl monomyristate, poly(2-10)glyceryl monostearate, poly(2-10)glyceryl monoisostearate, poly(2-10)glyceryl monooleate, diglyceryl sesquioleate, poly(2-10)glyceryl diisostearate, poly(6-10)glyceryl distearate,diglyceryltriisostearate, poly (10) glyceryl tristearate, poly(10)glyceryl trioleate, poly(2)glyceryl tetraisostearate, decaglyceryl pentastearate, poly(6-10)glyceryl pentaoleate, poly(10)glyceryl heptastearate, decaglyceryl decastearate, poly(10)glyceryl decaoleate, concentrated poly(6)glyceryl pentaricinoleate, sucrose fatty acid ester, sucrose cocoate, alkyl glucoside, cocodimethyl amine oxide, lauryl dimethylamine oxide, dihydroxyethyl lauryldimethylamine oxide, stearyl dimethylamine oxide, oleyl dimethyl amine oxide and polyoxyethylene cocoalkyl dimethyl amine oxide;

natural surface active agents, such as saponin, lecithin, soybean phospholipid, hydrogenated soybean phospholipid, soybean lysophospholipid, hydrogenated soybean lysophospholipid, egg yolk lecithin, hydrogenated egg yolk lysophosphatidylcholine, phosphatidylcholine, phosphatidylethanolamine, phsphatidylserine, sphingophospholipid, sphingomyelin, ganglioside, bile acid, cholic acid, deoxycholic acid, sodium cholate, sodium deoxycholate, spiculisporic acid, rhamnolipid, trehalose lipid, sophorolipid, mannosyl erythritol lipid, surfactin and its salt;

ultraviolet absorbing agents, e.g., p-aminobenzoic acid, p-aminobenzoic acid derivatives, such as ethyl p-aminobenzoate, glyceryl p-aminobenzoate, amyl p-dimethylaminobenzoate and 2-ethylhexyl p-dimethylaminobenzoate, cinnamic acid derivatives, such as benzyl cinnamate, glyceryl di-p-methoxycinnamate mono-2-ethylhexanoate, methyl 2,4-diisopropylcinnamate, ethyl 2,4-diisopropylcinnamate, potassium p-methoxycinnamate, sodium p-methoxycinnamate, isopropyl p-methoxycinnamate, 2-ethylhexyl p-methoxycinnamate, 2-ethoxyethyl p-methoxycinnamate and ethyl p-ethoxycinnamate, urocanic acid, urocanic acid derivatives, such as ethyl urocanate, benzophenone derivatives, such as 2,4-dihydroxybenzophenone, 2,2',4,4'-tetrahydroxybenzophenone, 2-hydroxy-4-methoxy-5-sulfobenzophenone sodium, 2-hydroxy-4-methoxybenzophenone-5-sulfonic acid, 2-hydroxy-4-methoxybenzophenone, 2,2'-dihydroxy-4,4'-dimethoxybenzophenone and 2,2'-dihydroxy-4,4'-dimethoxy-5-sulfobenzophenone sodium, salicylic acid derivatives, such as ethylene glycol salicylate, 2-ethylhexyl salicylate, phenyl salicylate, benzyl salicylate, p-tert-butylphenyl salicylate, homomentyl salicylate and 3,3,5-trimethylcyclohexyl salicylate, 2-(2'-hydroxy-5'-methoxyphenyl)benzotriazole, and 4-tert-butyl-4'-methoxybenzoylmethane;

powders and coloring materials, e.g., natural dyes, such as kaolin, silicic acid anhydride,aluminum magnesiumsilicate, sericite, talc, boron nitride, mica, montmorillonite, ramie cellulose powder, wheat starch, silk powder, corn starch, nitro dye, azo dye, nitroso dye, triphenylmethanedye, xanthene dye, quinoline dye, anthraquinone dye, indigo dye, pyrene dye, phthalocyanine dye, flavonoid, quinone, porphyrin, water-soluble annatto, cuttlefish ink powder, caramel, guaiazulene, gardenia blue, gardenia yellow, cochineal, shikonin, copper chlorophyllin sodium, paprika color, safflower red, safflower yellow, laccaic acid and riboflavin butyric acid ester, carbon black, yellow iron oxide, black iron oxide, red iron oxide, Prussian blue, ultramarine, zinc oxide, chromium oxide, titanium oxide, black titanium oxide, zirconium oxide, chromium hydroxide, alumina, magnesium oxide, barium sulfate, aluminum hydroxide, calcium carbonate, lithium cobalt titanate, manganese violet, and pearl pigment;

plant extracts, such as angelica extract, gambir extract, avocado extract, hydrangea extract, *Gynostemma pentaphyllum* extract, althea extract, arnica extract, oil-soluble arnica extract, almond extract, aloe extract, styrax resin extract, ginkgo extract, nettle extract, orris extract, fennel extract, turmeric extract, rose fruit extract, echinacea leaf extract, scutellaria root extract, phellodendron bark extract, Japanese coptis extract, barley extract, okura extract, hypericum extract, oil-soluble hypericum extract, white nettle extract, oil-soluble white nettle extract, restharrow extract, watercress extract, orange flower water, persimmon tannin, pueraria root extract, Japanese valerian extract, cattail extract, chamomile extract, oil-soluble chamomile extract, chamomile distillate, oat extract, carrot extract, oil-soluble carrot extract, carrot oil, *Artemisia capillaris* extract, glycyrrhiza extract, glycyrrhiza extract powder, glycyrrhiza flavonoid, cantharis tincture, raspberry extract, kiwi extract, cinchona extract, cucumber extract, apricot kernel extract, quince seed extract, gardenia extract, *Sasa Albo-marginata* extract, *Sophora* root extract, walnut shell extract, clematis extract, black sugar extract, chlorella extract, mulberry bark extract, cinnamon bark extract, gentian extract, geranium herb extract, black tea extract, nuphar extract, burdock root extract, oil-soluble burdock root extract, wheat germ extract, hydrolyzed wheat powder, rice bran extract, fermented rice bran extract, comfrey extract, asiasarum root extract, saffron extract, saponaria extract, oil-soluble sage extract, crataegus fruit extract, zanthoxylum fruit extract, shiitake extract, shiitake mushroom extract powder, rehmannia root extract, lithospermum root extract, oil-soluble lithospermumroot extract, perillaherb extract, Japanese linden extract, oil-soluble Japanese linden extract, filipendula extract, peony root extract, job's tears extract, ginger extract, oil-soluble ginger extract,
ginger tincture, calamus rhizome extract, birch extract, oil-soluble birch extract, birch sap, honeysuckle extract, horsetail extract, oil-soluble horsetail extract, scordinine, stevia extract, ivy extract, crataegus extract, sambucus extract, juniper extract, yarrow extract, oil-soluble yarrow extract, peppermint extract, sage extract, oil-soluble sage extract, sage water, mallow extract, celery extract, cnidium rhizome extract, cnidium rhizome water, swertia herb extract, soy extract, jujube extract, thymeextract, green tea extract, tea leaf dry distilled solution, tea seed extract, clove extract, citrus unshiu peel extract, camellia extract, centella extract, oil-soluble walnut extract, duku extract, terminalia extract, Japanese angelica root extract, oil-soluble Japanese angelica root extract, Japanese angelica root water, calendula extract, oil-soluble calendula extract, soy milk powder, peach seed extract, bitter orange peel extract, houttuynia extract, tomato extract, tormentilla extract, natto extract, ginseng extract, oil-soluble ginseng extract, garlic extract, wild rose extract, oil-solublewildroseextract, malt extract, malt root extract, *Ophiopogonis tuber* extract, parsley extract, barley leaf juice concentrate, peppermint distillate, witch hazel distillate, witch hazel extract, rose extract, pellitory extract,
Isodonis extract, loquat leaf extract, oil-soluble loquat leaf extract, coltsfoot extract, hoelen extract, butcher broom extract, butcher broom extract powder, grape extract, grape leaf extract, grape water, hayflower extract, sponge gourd extract, sponge gourd solution, safflower extract, oil-soluble linden extract, linden water, paeonia extract, hop extract, oil-soluble hop extract, pine extract, thistle extract, horse chestnut extract, oil-soluble horse chestnut extract, mukurossi peel extract, balmmint extract, sweet clover extract, peach leaf extract, oil-soluble peach leaf extract, bean sprouts extract, cornflower extract, cornflower distillate, eucalyptus extract, saxifrage extract, lily extract, coix extract, oil-soluble coix seed extract, mugwort extract, Japanese mugwort water, lavender extract, lavender water, apple extract, ganoderma extract, lettuce extract, Chinese milk vetch extract, rose water, rosemary extract, oil-soluble rosemary extract, Romanchamomile extract and burnet extract;

amino acids and peptides, such as glycine, valine, leucine, isoleucine, serine, threonine, phenylalanine, tyrosine, tryptophan, cystine, cysteine, methionine, hydroxyproline, aspartic acid, asparagine, glutamic acid, glutamine, histidine, γ-aminnobutyric acid, DL-pyrrolidonecarboxylic acid, ε-aminocaproic acid, hydrolyzed elastin, water-soluble elastin, hydrolyzed collagen, water-soluble collagen, casein, glutathione, wheat peptide and soybean peptide;

vitamins and substances having vitamin-like actions, e.g., vitamin A, such as retinol, retinal, retinoic acid, retinol acetate and retinol palmitate, carotenoids, such as α-carotene, β-carotene, γ-carotene, δ-carotene, lycopene, zeaxanthin, cryptoxanthin, echinenone and astaxanthin, vitamin B1, such as thiamines, vitamin B2, such as riboflavin, vitamin B6, such as pyridoxine, pyridoxal and pyridoxamine, vitamin B12, such as cyanocobalamin, folic acids, nicotinic acid, nicotinic acid amide, pantothenic acids, biotins, vitamin C, such as L-ascorbic acid, sodium L-ascorbate, L-ascorbyl stearate, L-ascorbyl palmitate, L-ascorbyl dipalmitate, L-ascorbyl tetraisopalmitate, disodium L-ascorbic acid sulfate, magnesium L-ascorbate, ascorbyl-2-phosphate, sodium L-ascorbyl-2-phosphate, magnesium L-ascorbyl phosphate, L-ascorbic acid-2-glucoside, sodium palmitic acid ascorbyl phosphate and sodium 2-hexyldecanoic acid ascorbyl phosphate, vitamin D, such as ergocalciferol and cholecalciferol, vitamin E, such as d-α-tocopherol, DL-α-tocopherol, dl-α-tocopherol acetate, dl-α-tocopherol succinate, β-tocopherol, γ-tocopherol, d-δ-tocopherol, sodium tocopheryl phosphate, tocopheryl dimethyl glycinate and its salt, vitamin K, carnitine, ferulic acid, γ-oryzanol, α-lipoic acid, and orotic acid;

antiseptics, such as benzoic acid, sodiumbenzoate, undecylenic acid, salicylic acid, sorbic acid, potassium sorbate, dehydroacetic acid, sodiumdehydroacetate, isobutyl parahydroxybenzoate, isopropyl parahydroxybenzoate, ethyl parahydroxybenzoate, butyl parahydroxybenzoate, propyl parahydroxybenzoate, benzyl parahydroxybenzoate, methyl parahydroxybenzoate, sodium methyl parahydroxybenzoate, phenoxyethanol, photosensitizing dye No. 101, photosensitizing dye No. 201 and photosensitizing dye No. 401;

antioxidants, such as butylhydroxyanisole, butylhydroxytoluene, propyl gallate, erysorbic acid, sodium erysorbate, p-hydroxyanisole and octyl gallate;

sequestering agents, e.g., metal ionic compounds, such as trisodium hydroxyethyl ethylenediamine triacetate, edetic acid, disodium edetate, trisodium edetate, tetrasodium edetate, sodium citrate, gluconic acid, phytic acid, sodium polyphosphate and sodium metaphosphate;

moisture-retaining agents, such as hyaluronic acid, sodium hyaluronate, sodium chondroitin sulfate, sodium lactate, sodium pyrrolidonecarboxylate, betaine, lactic acid bacteria culture solution, yeast extract and ceramide;

anti-inflammatory agents, such as glycyrrhizinic acid, trisodium glycyrrhizinate, dipotassium glycyrrhizinate, monoammonium glycyrrhizinate, β-glycyrrhetinic acid, glyceryl glycyrrhetinate, stearyl glycyrrhetinate, lysozyme chloride, hydrocortisone and allantoin;

pH adjustors, such as sodium hydroxide, potassium hydroxide and triethanolamine;

salts, such as sodium chloride, potassium chloride, magnesium chloride and sodium sulfate;

α-hydroxy acids, such as citric acid, glycolic acid, tartaric acid and lactic acid;

whitening agents, such as arbutin, α-arbutin and placental extract;

essential oils, such as angelica oil, ylang-ylang oil, elemi oil, chamomile oil, Romanchamomile oil, cardamom oil, calamus oil, galbanum oil, camphor oil, carrot seed oil, clary sage oil, clove oil, cinnamon oil, coriander oil, cypress oil, sandalwood oil, cedarwood oil, citronella oil, cinnamon leaf oil, jasmine absolute, juniper berry oil, ginger extract, spearmint oil, sage oil, cedar oil, geranium oil, thyme oil, tea tree oil, nutmeg oil, Ti-tree oil, neroli oil, pine oil, basil oil, mint oil, patchouli oil, palmarosa oil, fennel oil, petigrain oil, black pepper oil, frankincense oil, vetiver oil, peppermint oil, bergamot oil, benzoin oil, bois de rose oil, majoran oil, myrrh oil, melissa oil, eucalyptus oil, ravensara oil, lavandine oil, lavender oil, linden oil, rose oil, rosewood oil, rosemary oil and lovage oil;

terpenes, such as pinene, terpinene, terpinolene, myrcene and longifolene; and

others, such as perfume and water.

For the hair nourishing cosmetic of the present invention, the above-mentioned ingredients which are generally employable for hair nourishing cosmetics can be used, and in addition to them, the existing cosmetic ingredients can be further used. For example, there can be used all the cosmetic ingredients described in The Japanese Standards of Cosmetic Ingredients 2nd edition (edited by Pharmaceutical and Medical Device Regulatory Science Society of Japan and published by Yakuji Nippo, Ltd. (1984)), The Japanese Cosmetic Ingredients Codex (edited by Ministry of Health and Welfare, Pharmaceutical Affairs Bureau, Examination Division and published by Yakuji Nippo, Ltd. (1993)), Supplement to The Japanese Cosmetic Ingredients Codex (edited by Ministry of Health and Welfare, Pharmaceutical Affairs Bureau, Examination Division and published by Yakuji Nippo, Ltd. (1993)), The Comprehensive Licensing Standards of Cosmetics by Category (edited by Ministry of Health and Welfare, Pharmaceutical Affairs Bureau, Examination Division and published by Yakuji Nippo, Ltd. (1993)), The Japanese Cosmetic Ingredients Codex by Category (edited by Ministry of Health and Welfare, Pharmaceutical Affairs Bureau, Examination Division and published by Yakuji Nippo, Ltd. (1997)), Keshouhin Genryou Jiten (Dictionary of Cosmetic Ingredients) (Nikko Chemicals. , Co. Ltd. (1991)), Atarashii Keshouhin Kinou Sozai 300 (300 Latest Cosmetic Functional Materials) (CMC Publishing Co., Ltd. (2002)), etc.

The hair nourishing cosmetic of the present invention can be prepared by using the above-mentioned ingredients in given amounts and dissolving, mixing or dispersing them in a conventional manner according to its embodiment.

The hair nourishing cosmetic of the present invention can be used after preparing it in various formulations, such as hair tonic, hair lotion, hair treatment, hair cream, hair conditioner, shampoo, rinse, hair gel, hair mist and hair foam, in accordance with a conventional process. Furthermore, for the purpose of nourishing hair, growing hair and/or preventing hair loss, the hair nourishing cosmetic of the present invention is used by applying or spraying it to the desired limited part (scalp) in accordance with the formulation.

### EXAMPLES

The present invention is further described with reference to the following examples, but it should be construed that present the invention is in no way limited to those examples.

[Synthesis Example 1] Synthesis of ubiquinol In 300ml of hexane, 30 g of ubiquinone (MW863.36) was dissolved. With stirring the solution at room temperature, 300 ml of a 10% (w/v) sodium hyposulfite solution was poured into the solution, and they were further stirred for 2 hours at room temperature. The whole amount of the resulting mixture was transferred into a separatory funnel, and a hexane layer was separated and obtained. The remaining aqueous layer was further extracted with 50 ml of hexane twice, and the extract was mixed with the hexane layer previously obtained. The resulting hexane layer was washed with 50 ml of a degassed saturated salt solution six times to obtain a transparent hexane layer. After hexane was removed under reduced pressure, purging with nitrogen was carried out for one day and night to obtain 29.5 g of a solid of cream color.

### [Synthesis Example 2] Synthesis of tetrapotassium ubiquinol-1,4-diphosphate

In 10 ml of pyridine, 3 g of the solid obtained in Synthesis Example 1 was dissolved. In a freezingmixture (salt/ice) bath (-15°C), to the resulting solution was dropwise added a solution obtained by dissolving 3.19 g of phosphorus oxychloride in 5 ml of pyridine, and they were stirred for 30 minutes with cooling. Thereafter, the temperature of the resultingmixture was returned to normal temperature, and the mixture was further stirred for 2 hours. After the solution was removed under reduced pressure, the resulting oily matter was suspended in 300 ml of diethyl ether, and 150 ml of a saturated salt solution was added. The whole amount of the resulting mixture was transferred into a separatory funnel, and the mixture was shaken and stirred and then allowed to stand still to obtain an ether layer. To the resulting ether layer was added 120 ml of dilute hydrochloric acid (concentrated hydrochloric acid:water = 1:2) to wash the ether layer, and then anhydrous magnesium sulfate was added to perform dehydration. The solution was removed under reduced pressure to obtain 2.5 g of a yellow oily matter. To it was added 30 ml of methanol to dissolve the oilymatter, and then a solution obtained by dissolving 0.547 g of potassium hydroxide in 2 g of methanol was dropwise added. The organic solvent was removed under reduced pressure, and purging with nitrogen was carried out for one day and night to obtain 2.63 g of a yellow fine powder. The resulting yellow fine powder was subjected to NMR measurement and mass spectrometry, and as a result, it was identified as potassium ubiquinol-1,4-diphosphate.

[NMR]
<¹H-NMR>
1.4-1.65 ppm (11H), 1. 8-1. 9 ppm (9H), 1.9-2.0ppm (10H), 2.2-2.4 ppm (3H), 3.8-4.0 ppm (6H), 4.95-5.1 ppm (10H)
<³¹P-NMR>
1.0-1.2 ppm
<Method>
Apparatus: Burker Advance-500
Solvent: D₂O.

(Mass spectrometry)
FAB-MS(-): 1023 (=[M-H]⁻)
<Method>
Direct introduction FAB-MS method
Apparatus: JEOL JMS-SX102A
FAB matrix: glycerin
Scanning range: m/Z 50-200.

### [Example 1]

To a solution A which had been previously prepared by blending ingredients in accordance with the composition described in the column of "Cosmetic 1" of the following Table 1, then stirring and dissolving them, a solution B which had been separately prepared by homogeneously dissolving ingredients was slowly added, and they were homogeneously mixed and stirred to obtain a cosmetic 1.

### [Comparative Example 1]

A cosmetic 2 was obtained in the same manner as in Example 1, except that a solution A and a solution B were each prepared in accordance with the composition described in the column of "Cosmetic 2" of Table 1. The cosmetic 2 is a control agent containing no ubiquinone derivative.

Properties of the cosmetics 1 and 2 obtained in Example 1 and Comparative Example 1 were both stable, and when the cosmetics 1 and 2 were applied to the skin, feelings in use were equivalent to each other.

[Table 1]

**Table 1**

| | | Cosmetic 1 | Cosmetic 2 |
|---|---|---|---|
| A | Olive oil | 0.5 | 0.5 |
| | Isopropyl myristate | 0.5 | 0.5 |
| | Isopropyl methylphenol | 0.05 | 0.05 |
| | Ethanol | 55.0 | 55.0 |
| B | Tetrapotassium ubiquinol-1,4-diphosphate | 1.0 | |
| | Glycerin | 1.0 | 1.0 |
| | Methylparaben | 0.1 | 0.1 |
| | Perfume | 0.1 | 0.1 |
| | Purified water | residue | residue |

| | | | |
|---|---|---|---|
| (Each value is percentage by mass given when the total amount is 100%.) | | | |

### [Test Example 1] Test of human hair growth promotion effect

10 male subjects of thirties to forties who realized the decline of hair growth had their hair at the top of the head shorn off in a shape of a circle having a diameter of about 7 mm. After one day and after three days from the hair shearing, the hair growing rates of hairs (about 30 to 40 hairs) at the objective part were determined by the method of Hayashi, et al. (British Journal of Dermatology, Vol. 125, p. 123, 1991), and a mean value (A) was calculated. Next, to the objective part and its circumference of each subject, about 3 ml of each of the cosmetics (cosmetic 1 and cosmetic 2) prepared in Example 1 and Comparative Example 1 was applied twice (morning and evening) a day, followed by well massaging. After 3 months from the beginning of the test, measurement of the hair growing rates at the same part was carried out in the same manner, and a mean value (B) was calculated. The ratio of the mean value after use of the hair nourishing cosmetic to that before use of it, (B)/(A), was regarded as a hair growth promotion effect, and the results are set forth in Table 2.

[Table 2]

**Table 2**

| Result of human hair growth promotion effect test | |
|---|---|
| | Hair growth promotion effect |
| Cosmetic 1 | 1.22 |
| Cosmetic 2 | 1.04 |

### [Test Example 2] Organoleptic test

To each of the heads of 20 male subjects of thirties to forties who were observed to have the decline of hair growth, the samples were each applied twice (morning and evening) a day over a period of continuous 6 months, and the effect was evaluated. The number of subjects who answered "the hair became bristly, or bristles increased" and the number of subjects who answered "the hair loss decreased" are set forth in Table 3 as an indication of the hair growing effect and as an indication of the hair loss prevention effect, respectively.

[Table 3]

**Table 3**

| Result of organoleptic test | | |
|---|---|---|
| | Hair nourishing effect | Hair loss prevention effect |
| Cosmetic 1 | 7 | 9 |
| Cosmetic 2 | 3 | 2 |

### [Test Example 3] Hair regrowth promotion test using mouse

15 male C3H/HeN S1c 7-week old mice in the resting stage of the hair cycle had their hair at the back part shorn off carefully with an electric hair clipper and an electric shaver. These mice were divided into three groups each containing 5 mice. To the skin of the shorn part of each mouse in one group, 0.1 ml of a test sample shown in the following Table 4 was applied once a day, and to the skin of the shorn part of each mouse in another group, a base was applied in the same amount. The residual group in which nothing had been applied was regarded as an untreated control.

After 20 days from the beginning of the experiments, the back part of each mouse was photographed. By the use of an image analyzing software (Image-J), a ratio of the area where the hair had regrown to the whole area of the hair shornpart was calculated, and the resulting ratio was regarded as a hair regrowth ratio. The hair regrowth ratio of each mouse was calculated, and statistical analysis of the hair regrowth ratio of each group was carried out. The results are shown in Fig. 1 and the following Table 5. In the group in which the test sample containing tetrapotassium ubiquinol-1,4-diphosphate had been administered, a tendency of enhancement of the hair regrowth ratio was observed.

**Table 4**

| Table 4 (Test sample) | |
|---|---|
| Base | Macrogol 400:Macrogol 4000 = 9:1 |
| Test sample | Sample obtained by adding 50 mmol/g of tetrapotassium ubiquinol-1,4-diphosphate to the above base and sufficiently kneading them. |

Table 5

**Table 5 (Hair regrowth ratio)**

| Group | | Control | Base | Tetrapotassium ubiquinol-1,4-diphosphate (test sample) |
|---|---|---|---|---|
| Number of data (n) | | 5 | 5 | 5 |
| Hair regrowth ratio (%) | Average | 52.96 | 41.67 | 58.45 |
| | SD | 23.69 | 21.15 | 21.03 |

## Claims

1. A hair nourishing cosmetic comprising a ubiquinone derivative represented by the following formula (1) and/or its salt: 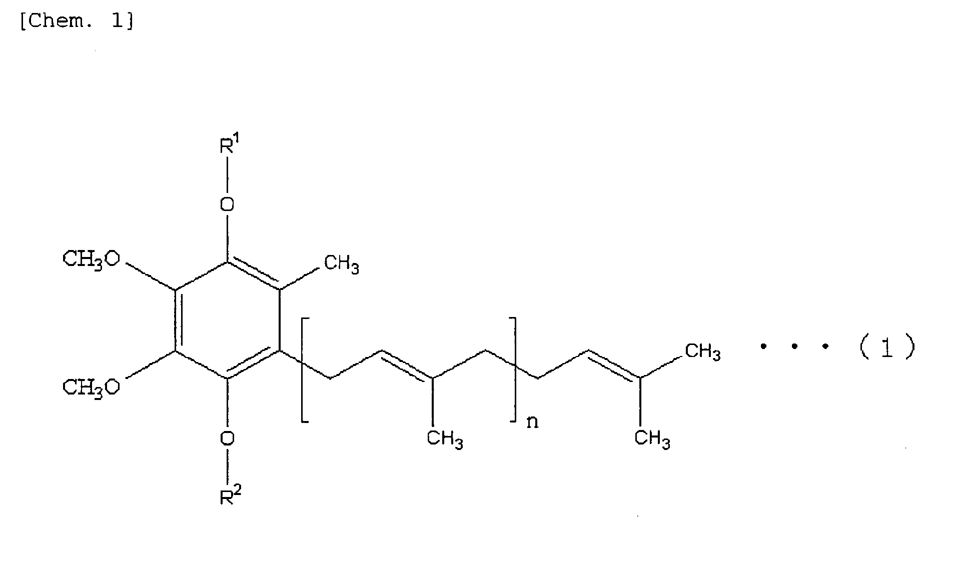 wherein R¹ and R² are each independently a hydrogen atom or a phosphoric acid group, at least one of R¹ and R² is a phosphoric acid group, and n is an integer of 1 to 9.

2. The hair nourishing cosmetic as claimed in claim 1, wherein the ubiquinone derivative and/or its salt is contained in a concentration of 0.0005 to 50% by mass.

3. The hair nourishing cosmetic as claimed in claim 1 or 2, wherein in the formula (1), both of R¹ and R² are phosphoric acid groups, and n is an integer of 5 to 9.
